# EUROPÄISCHE PATENTSCHRIFT

(11) **EP 0 583 712 B1**
(45) Veröffentlichungstag und Bekanntmachung des Hinweises auf die Patenterteilung: **23.04.1997**
(21) Anmeldenummer: 93112731.0
(22) Anmeldetag: 09.08.1993
(51) Int. Cl.: C07C 31/20, C07C 39/16, C07C 37/20

(54) **Verfahren zur Herstellung von Bisphenolen**
Process for the preparation of bisphenols
Procédé pour la préparation de bisphénols

(30) Priorität: 20.08.1992 DE 4227520
(43) Veröffentlichungstag der Anmeldung: 23.02.1994
(73) Patentinhaber: BAYER AG, 51368 Leverkusen (DE)
(72) Erfinder: Dr. Berg, Klaus, D-47798 Krefeld (DE); Dr. Buysch, Hans-Josef, D-47809 Krefeld (DE); Dr. Eitel, Alfred, D-41539 Dormagen (DE); Dr. Fennhoff, Gerhard, D-47877 Willich (DE); Dr. Immel, Otto, D-47803 Krefeld (DE); Dr. Pakull, Ralf, D-50765 Köln (DE); Dr. Wehrle, Bernhard, D-40764 Langenfeld (DE); Dr. Wulff, Claus, D-47800 Krefeld (DE)

(56) Entgegenhaltungen:
- EP-A- 0 313 165
- EP-A- 0 342 758
- US-A- 3 634 341
- US-A- 4 391 997
- US-A- 5 210 328

## Beschreibung

Vorliegende Erfindung betrifft ein Verfahren zur Herstellung von Bisphenolen aus Aceton und Phenolen in Gegenwart von mit Alkyl-SH-Gruppen modifizierten sulfonsauren Ionenaustauscherharzen bei dem man dem Gemisch aus Phenol und Aceton 0,6 bis 5 Gew.-% H₂O vor der Umsetzung zugibt.

Die Kondensation von Phenolen und Carbonylverbindungen zur Bildung von Bisphenolen unter Verwendung verschiedener Katalysatoren wie Chlorwasserstoffsäure (US-PS 2.182.308 und 2.191.831), Bortrifluorid (CA 58, 3338c), Perchlorsäure (CA 60, 1626h), Benzolsulfonsäure (CA 59, 511h) und unter Verwendung der verschiedensten Kationenaustauscherharze (z.B. GB 842.209, 849.565 und 883.391) ist bekannt. Auch der Zusatz schwefelhaltiger Verbindungen als Cokatalysatoren ist bekannt. Die Verwendung von Thioglykolsäure und 3-Mercaptopropionsäure ist z.B. in US-PS 2.468.982 und 2.623.908 beschrieben. Der Zusatz von Alkylmercaptanen ist z.B. in US-PS 2 775 620, der Zusatz von Schwefelwasserstof ist z.B. in CA 58, 1403e beschrieben. Die bekannten schwefelhaltigen Katalysatoren können in der betrieblichen Praxis zu erheblichen Korrosionsschäden führen. Die auf diese Weise hergestellten Bisphenole sind mit Katalysatorspuren verunreinigt und enthalten einen hohen Anteil an Nebenprodukten, z.B. zum gewünschten Bisphenol isomere Kondensationsprodukte (CA = Chemical Abstracts).

Demgegenüber gestattet die Synthese von Bisphenolen z.B. gemäß der DE-A 3.619.450 oder DE-A 3.727.641 unter Verwendung von Ionenaustauschern, welche mit Alkyl-SH-Gruppen modifiziert worden sind, die Herstellung und Isolierung hochreiner Bisphenole.

Bei der Herstellung von Bisphenolen aus Monophenolen und Carbonylverbindungen wie Aldehyden und Ketonen gemäß z.B. DE-A 3.619.450 und DE-A 3.727.641 ist jedoch ein starkes Abnehmen der Katalysatorreaktivität und Selektivität bereits nach relativ kurzen Produktionsphasen zu verzeichnen. Das eingesetzte Katalysatorsystem muß dann entweder regeneriert oder sogar vollständig erneuert werdend Dies führt zu Produktionsstillständen und zusätzlichem Mehraufwand in der Wartung der Produktionsanlagen, also zu völlig unzureichenden Raum-Zeit-Ausbeuten.

Es wurde nun gefunden, daß die katalytische Aktivität der modifizierten Ionenaustauscherharze wesentlich länger erhalten bleibt, wenn dem Eduktgemisch aus Aceton und Phenolen Wasser in einer Menge von 0,6 bis 5 Gew.-% bezogen auf das Eduktgemisch zugesetzt wird Beim erfindungsgemäßen Verfahren wird die Selektivität der Umsetzung zu den Bisphenolen gesteigert und damit deren Reinheit durch die Wasserzugabe verbessert.

Dies war nicht vorherzusehen, zumal z.B. aus Angew. Chem. 75, S. 662 (1963) bekannt ist, daß Ionentauscherharze nur in trockenem Zustand für eine Bisphenolsynthese brauchbar sind.

Aus z.B. der EP-A 319 327 ist bekannt, daß die Austauscherharze entweder von Beginn an trocken sein sollen oder aber durch trockene Eduktgemische bei kontinuierlicher Arbeitsweise nach und nach getrocknet werden müssen, will man annehmbare Umsätze erzielen und möglichst isomerenfreie Produkte erhalten. Ähnliches ist auch für Ionenaustauscherharze, die mit Alkyl-SH-Gruppen modifiziert sind, bekannt (z.B. EP-A 319 327, EP-A 49 411, EP-A 268 318). Aus der US-A 3 634 341 ist bekannt, daß mit Aminothiolen oder Thiazolidinen teilneutralisierte Austauscherharze sogar gegenüber Wasser empfindlich sein sollen.

So kann man z.B. gemäß der EP-A 442 122 das während der Reaktion gebildete Wasser möglichst rasch und wirksam aus dem Reaktionsgemisch mit Hilfe von Pervaporationsmembranen entfernen um störende Nebenreaktionen und -effekte zu vermeiden.

Nach dem erfindungsgemäßen Verfahren erhält man durchaus befriedigende bis ausgezeichnete Umsätze bei erhöhter Selektivität. Dies ist besonders erwünscht, weil die nicht in p,p'-Isomere umlagerbaren Nebenprodukte wie Chromane und Indane kaum gebildet werden. Die Isolierung von reineren Bisphenolen ist erleichtert.

Die durch Wasserzusätze erreichbare Standzeit der mit Mercaptogruppen modifizierten Ionenaustauscherharze beträgt ein mehrfaches der bei Verwendung trockener Edukte. So kann in der Regel schon nach einer Betriebszeit von wenigen 100 h eine deutliche Schädigung des Katalysators festgestellt werden, wird trockenes Edukt eingesetzt. Diese Schädigung ist an einer Abnahme von Mercaptogruppen im Harz erkennbar. Bei Verwendung wasserhaltiger Edukte wird auch nach einigen 1000 h praktisch der gleiche Mercaptogehalt wie zu Beginn beobachtet. Diesen Befund bestätigt die gleichbleibende Aktivität des Katalysators.

Gegenstand der vorliegenden Erfindung ist also ein Verfahren zur Herstellung von 4,4'-Dihydroxydiphenylpropanen aus Phenolen und Aceton in Gegenwart von mit Alkyl-SH-Gruppen modifizierten sulfonsauren Ionenaustauschern, dadurch gekennzeichnet, daß man dem Eduktgemisch aus Aceton und Phenolen 0,6 bis 5 Gew.-% Wasser vor der Umsetzung zusetzt.

Geeignete Ausgangsprodukte für das erfindungsgemäße Verfahren sind Phenole wie o-Kresol, 2,6-Dimethylphenol, o-Ethylphenol, i-Isopropylphenol, o-tert.-Butylphenol, o-Phenylphenol, besonders aber Phenol und Ketone wie Aceton.

Geeignete Ionenaustauscherharze sind sulfonierte vernetzte Styrolpolymerisate und Phenolharze. Bevorzugt sind gelförmige mit 2 bis 6 Gew.-%, besonders bevorzugt 2 bis 4 Gew.-% Divinylbenzol, bezogen auf Styrol vernetzte sulfonierte Styrolpolymerisate, die auch mehr als 1 Sulfonsäuregruppe pro Styrolkern enthalten können und in denen die Sulfonsäuregruppen zu 1 bis 30 %, bevorzugt 3 bis 25 %, besonders bevorzugt 3 bis 15 % z.B. mit Aminoalkylmercaptanen, Thiazolidinen oder Aminothiophenolen neutralisiert sind (z.B. DE-A 3 619 450, 3 727 641, EP-CA 319 327, EP-CA 268 318, DE-A 2 733 537). Bevorzugt sind Aminoalkylmercaptane und Thiazolidine, besonders bevorzugt Cysteamin und Dimethylthiazolidin. Die Teilneutralisation der Sulfonsäuren wird nach bekannten Verfahren vorgenommen (z.B. DE-A 3 619 450, DE-A 3 727 641, US-A 3 394 089).

Es können auch Mischungen von sulfonsauren und Alkyl-SH-Gruppen (ionisch oder kovalent gebunden) enthaltende Ionenaustauscherharse verwendet werden.

Die Kondensation von Aceton mit den Phenolen erfolgt unter an sich bekannten Bedingungen bei 50 bis 100°C, bevorzugt 55 bis 90°C, besonders bevorzugt 55 bis 85°C, mit Katalysatorbelastungen von 0,05 bis 2,0 kg Eduktgemisch pro 1 kg Harz und h, bevorzugt 0,10 bis 1,0, besonders bevorzugt 0,1 bis 0,5 kg/l/h bei Molverhältnissen von Aceton zu Phenol von 1:25 bis 1:4, bevorzugt 1:22 bis 1:6, besonders bevorzugt 1:20 bis 1:8.

Der Zusatz an Wasser zum Eduktgemisch beträgt 0,6 bis 5 Gew.-%, bezogen auf das Eduktgemisch von Aceton und Phenol, bevorzugt 1,0 bis 4,1 Gew.-%, besonders bevorzugt 1,2 bis 2,9 Gew.-%.

### Beispiel 1

50 ml eines getrockneten mit 2 % Divinylbenzol vernetzten sulfonierten Styrolpolymerisates (Lewatit SC 102) wurden in Phenol vorgequollen und dann in phenolischer Suspension unter Rühren mit 3,3 g in Phenol gelöstem Cysteamin tropfenweise versetzt und 24 h weitergerührt. Diese Suspension wurde in einen beheizbaren Rohrreaktor gefüllt und dazu 25 g/h einer Eduktmischung aus Phenol mit 3,8 Gew.-% Aceton bei 65°C hinzugepumpt, wobei dem Eduktgemisch
a) während 200 h Laufzeit kein Wasser und
b) während 300 h Laufzeit 1 % Wasser
zugesetzt wurde. Das Ergebnis zeigt Tabelle 1.

**Tabelle 1**

| | Umsatz Aceton | Selekt. für p,p'-BPA | Selektivität* |
|---|---|---|---|
| a | 98-99 % | 94,2 % | 0,27 % |
| b | 95-96 % | 94,7 % | 0,12 % |

| | | | |
|---|---|---|---|
| * nicht umlagerbare Nebenprodukte | | | |

Man erhält also eine merkliche Verbesserung der Selektivität um 0,5 % und eine Abnahme der unbrauchbaren, nicht isomerisierbaren (umlagerbaren) Nebenprodukte um mehr als die Hälfte bei fast gleichem Acetonumsatz.

### Beispiel 2

Mit dem Katalysator aus Beispiel 1 wurde unter sonst gleichen Bedingungen weiter gearbeitet mit der Ausnahme, daß die Temperatur auf 70°C angehoben wurde und
a) während 250 h 1 % Wasser und
b) während 250 h 2 % Wasser
zugesetzt wirden. Das Ergebnis gleicht dem von Beispiel 1. Man erhält beim Übergang von 1 % auf 2 % Wasserzugabe zum Eduktgemisch einen marginalen Umsatzrückstand von 3 bis 4 %, jedoch eine Selektivitätsanhebung um 0,3 % und eine Reduzierung der unbrauchbaren Nebenprodukte um etwa 20 %.

### Beispiel 3

Nach 1000 h Laufzeit ist keine Abnahme der Aktivität des Katalysators aus Beispiel 1 und 2 bei Zusatz von Wasser zu erkennen.

Nach dieser Zeit wird der Katalysator mit Phenol gespült und mit Hilfe der NMR-Festkörperspektroskopie untersuchte Dabei wird gegenüber dem frischen Katalysator keine Abnahme des SH-Gruppengehaltes ermittelt.

Wird jedoch der gleiche Katalysator über 1000 h mit einem trockenen, d.h. ca. 0,1 % Wasser enthaltenden Eduktgemisch, belastet, so sinkt die Aktivität um etwa 25 % auf einen Acetonumsatz von 70 bis 75 % ab und im ¹H-NMR wird eine deutliche Abnahme des SH-Gruppengehalts um ca. 60 bis 70 % beobachtet.

## Patentansprüche

1. Verfahren zur Herstellung von 4,4-Dihydroxy-diphenylpropanen aus Aceton und Phenolen in Gegenwart von mit Alkyl-SH-Gruppen modifizierten sulfonsauren Ionenaustauscher, dadurch gekennzeichnet, daß man dem Eduktgemisch von Aceton und Phenolen 0,6 bis 5 Gew.-% Wasser zugibt.

## Claims

1. A method of preparing 4,4-dihydroxy-diphenyl-propanes from acetone and phenols in the presence of sulphonic acid ion exchangers modified with alkyl-SH groups, characterised in that 0.6 to 5 % by weight of water is added to the mixture of starting materials comprising acetone and phenols.

## Revendications

1. Procédé pour la préparation de 4,4'-dihydroxy-diphénylpropanes à partir d'acétone et de phénols en présence de résines échangeuses d'ions sulfonées modifiées par des groupes alkyl-SH, caractérisé en ce qu'on ajoute au mélange d'éduit d'acétone et de phénols de 0,6 à 5% en poids d'eau.
